# EUROPEAN PATENT SPECIFICATION

(11) **EP 2 884 983 B1**
(45) Date of publication and mention of the grant of the patent: **04.10.2017**
(21) Application number: 13750830.5
(22) Date of filing: 14.08.2013
(51) Int. Cl.: A61K 31/5517, A61P 35/00

(54) **BENZODIAZEPINES FOR TREATING SMALL CELL LUNG CANCER**
BENZODIAZEPINES ZUR BEHANDLUNG VON KLEINZELL-LUNGENKREBS
BENZODIAZEPINES POUR TRAITER LE CARCINOME PULMONAIRE À PETITES CELLULES

(30) Priority: 16.08.2012 US 201261683811 P; 23.04.2013 US 201361814886 P
(43) Date of publication of application: 24.06.2015
(73) Proprietor: GlaxoSmithKline LLC, Wilmington, DE 19808 (US)
(72) Inventor: BARBASH, Olena, I, Collegeville, Pennsylvania 19426 (US); TUMMINO, Peter, John, Collegeville, Pennsylvania 19426 (US)
(74) Representative: Shore, Andrew David
(86) International application number: PCT/US2013/054818
(87) International publication number: WO 2014/028547

(56) References cited:
- WO-A1-2011/054553
- WO-A1-2011/054845
- DECAUDIN D ET AL: "Peripheral benzodiazepine receptor ligands reverse apoptosis resistance of cancer cells in vitro and in vivo", CANCER RESEARCH 20020301 US, vol. 62, no. 5, 1 March 2002 (2002-03-01), pages 1388-1393, XP8164854, ISSN: 0008-5472
- MOODY T W ET AL: "CI-988 inhibits growth of small cell lung cancer cells", JOURNAL OF PHARMACOLOGY AND EXPERIMENTAL THERAPEUTICS 2001 US, vol. 299, no. 3, 2001, pages 1154-1160, XP8164860, ISSN: 0022-3565
- PANAGIS FILIPPAKOPOULOS ET AL: "Benzodiazepines and benzotriazepines as protein interaction inhibitors targeting bromodomains of the BET family", BIOORGANIC & MEDICINAL CHEMISTRY, vol. 20, no. 6, 1 March 2012 (2012-03-01), pages 1878-1886, XP55070757, ISSN: 0968-0896, DOI: 10.1016/j.bmc.2011.10.080

## Description

### Field of the Invention

The present invention relates to a benzodiazepine compound and its use in the treatment of cancer, particularly small cell lung cancer.

### Background of the Invention

Lung cancer is a disease characterized by uncontrolled cell growth in tissues of the lung. The main types of lung cancer are non-small cell lung cancer (NSCLC) and small cell lung cancer (SCLC). Small cell lung cancer although less common, is the most aggressive form of lung cancer with almost all cases being due to cigarette smoking. Small cell lung carcinoma usually starts as small cancerous cells in the bronchi but these grow very quickly to create large tumors, which spread rapidly to other parts of the body, such as the brain, liver and/or bone. Small cell lung carcinoma is typically treated with a combination of chemotherapy and radiation therapy.

Patent applications WO2011/054553 and WO2011054845 disclose the compound 2-[(4*S*)-6-(4-chlorophenyl)-1-methyl-8-(methyloxy)-4*H*-[1,2,4]triazolo[4,3-*a*][1,4]benzodiazepin-4-yl]-*N-*ethylacetamide as a BET family bromodomain inhibitor and describes therapeutic uses thereof.

### Summary of the Invention

In a first aspect of the present invention, there is provided 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof for use in the treatment of small cell lung cancer.

In a second aspect of the present invention, there is provided a method of treating small cell lung cancer in a subject in need thereof which comprises administering a therapeutically effective amount of 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof.

In a third aspect of the present invention, there is provided a pharmaceutical composition comprising 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof for use in the treatment of small cell lung cancer.

In a fourth aspect of the present invention, there is provided a combination pharmaceutical product comprising 2-[(4*S*)-6-(4-chlorophenyl)-1-methyl-8-(methyloxy)-4*H-*[1,2,4]triazolo[4,3-*a*][1,4]benzodiazepin-4-yl]-*N*-thylacetamide or a pharmaceutically acceptable salt thereof together with one or more further therapeutically active agents suitable for use in the treatment of small cell lung cancer.

### Description of the Drawings

Figure 1: Showing that the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide attenuates the growth/survival of SCLC cell lines and primary tumour models *in vitro.*
Figure 2: Showing that the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide induces cell death in a subset of SCLC cell lines.
Figure 3: Showing *in vivo* efficacy and pharmacodynamic activity for the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide in a SCLC mouse xenograft.

### Detailed Description of the Invention

The present invention relates to 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide, that is to say the compound of formula (I) 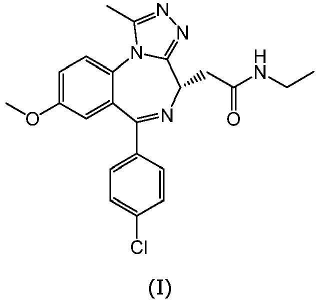 or a pharmaceutically acceptable salt thereof for use in the treatment of small cell lung cancer.

Suitable pharmaceutically acceptable salts are familiar to those skilled in the art (see Berge et al., J. Pharm. Sci., 66:1-19, (1977)) and specific examples of which are described further in WO2011/054553. Pharmaceutically acceptable salts can be stoichiometric or non-stoichiometric.

In one embodiment 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide is in the form of a free base.

It will be further appreciated that 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide or a pharmaceutically acceptable salt thereof could be in any suitable solvated (e.g. hydrated) and/or polymorphic forms thereof.

The compound 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide or a pharmaceutically acceptable salt thereof may be prepared according to procedures described in patent application WO2011/054553 or by similar methods thereto.

In one aspect of the invention there is provided a composition for treating small cell lung cancer in a subject in need thereof which comprises administering a therapeutically effective amount of 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof.

Also disclosed is a method of treating acute myelogenous leukemia in a subject in need thereof which comprises administering a therapeutically effective amount of 2-[(4*S*)-6-(4-chlorophenyl)-1-methyl-8-(methyloxy)-4*H*-[1,2,4]triazolo[4,3-*a*][1,4]benzodiazepin-4-yl]-*N-*ethylacetamide or a pharmaceutically acceptable salt thereof. In particular there is provided a method of treating a human patient with a diagnosis of relapsed and / or refractory acute myelogenous leukemia.

In one embodiment the subject in need thereof is a human subject.

As used herein, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

Further provided is the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof for use in the manufacture of a medicament for use in the treatment of small cell lung cancer. Also disclosed is the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof for use in the manufacture of a medicament for use in the treatment of acute myelogenous leukemia.

The compound 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide or a pharmaceutically acceptable salt thereof can also be used in the treatment of one or more cancers selected from brain cancer (gliomas), glioblastomas, Bannayan-Zonana syndrome, Cowden disease, Lhermitte-Duclos disease, breast cancer, inflammatory breast cancer, colorectal cancer, NUT-midline carcinoma, Wilm's tumor, Ewing's sarcoma, rhabdomyosarcoma, ependymoma, medulloblastoma, colon cancer, head and neck cancer, kidney cancer, non small cell lung cancer, liver cancer, melanoma, squamous cell carcinoma, ovarian cancer, pancreatic cancer, prostate cancer, sarcoma cancer, osteosarcoma, giant cell tumor of bone, thyroid cancer, lymphoblastic T-cell leukemia, chronic myelogenous leukemia, chronic lymphocytic leukemia, hairy-cell leukemia, acute lymphoblastic leukemia, acute myelogenous leukemia, chronic neutrophilic leukemia, acute lymphoblastic T-cell leukemia, plasmacytoma, immunoblastic large cell leukemia, mantle cell leukemia, multiple myeloma, megakaryoblastic leukemia, acute megakaryocytic leukemia, promyelocytic leukemia, mixed lineage leukaemia, erythroleukemia, malignant lymphoma, Hodgkins lymphoma, non-Hodgkins lymphoma (e.g. double-hit (Bcl2, Myc) lymphoma), lymphoblastic T-cell lymphoma, Burkitt's lymphoma, follicular lymphoma, neuroblastoma, bladder cancer, urothelial cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor) and testicular cancer.

In particular there is described the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof for use in the treatment of acute myelogenous leukemia (also know as acute myeloid leukemia or AML).

While it is possible that for use in therapy, 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide as well as pharmaceutically acceptable salts thereof may be administered as the raw chemical, it is common to present the active ingredient as a pharmaceutical composition with one or more pharmaceutically acceptable carrier(s), diluent(s) or excipient(s).

In a further aspect there is described a pharmaceutical composition comprising 2-[(4*S*)-6-(4-chlorophenyl)-1-methyl-8-(methyloxy)-4*H*-[1,2,4]triazolo[4,3-*a*][1,4]benzodiazepin-4-yl]-*N-*ethylacetamide or a pharmaceutically acceptable salt thereof for use in the treatment of one or more cancer type as disclosed herein. In a particular embodiment there is provided a pharmaceutical composition comprising 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide or a pharmaceutically acceptable salt thereof for use in the treatment of small cell lung cancer. Further, there is disclosed a pharmaceutical composition comprising 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide or a pharmaceutically acceptable salt thereof for use in the treatment of acute myelogenous leukemia.

Pharmaceutical compositions may be adapted for administration by any appropriate route, for example by the oral (including buccal or sublingual), rectal, inhaled, intranasal, topical (including buccal, sublingual or transdermal), vaginal or parenteral (including subcutaneous, intramuscular, intravenous or intradermal) route. In one embodiment the pharmaceutical composition is adapted for oral administration.

Suitable methods for formulating 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide or a pharmaceutically acceptable salt thereof include those described in patent application WO2011/054553 and / or the methods that are familiar to those skilled in the art, which are described in Remington: The Science and Practice of Pharmacy, 21st Edition 2006.

Pharmaceutical compositions may be presented in unit dose forms containing a predetermined amount of active ingredient per unit dose. Preferred unit dosage compositions are those containing a daily dose or sub-dose, or an appropriate fraction thereof, of an active ingredient. Such unit doses may therefore be administered more than once a day. Preferred unit dosage compositions are those containing a daily dose or sub-dose (for administration more than once a day), as herein above recited, or an appropriate fraction thereof, of an active ingredient.

A therapeutically effective amount of 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or pharmaceutically acceptable salt thereof will depend upon a number of factors including, for example, the age and weight of the animal, the precise condition requiring treatment and its severity, the nature of the formulation, and the route of administration, and will ultimately be at the discretion of the attendant physician or veterinarian. In the pharmaceutical composition, each dosage unit for oral or parenteral administration preferably contains from 0.01 to 3000 mg, more preferably 0.5 to 1000 mg, even more preferably 1.0 to 100mg of the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or pharmaceutically acceptable salt thereof calculated as the free base. Each dosage unit for nasal or inhaled administration preferably contains from 0.001 to 50 mg, more preferably 0.01 to 5 mg, calculated as the free base.

The compound 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide and pharmaceutically acceptable salts thereof can be administered in a daily dose (for an adult patient) of, for example, an oral or parenteral dose of 0.01 mg to 3000 mg per day or 0.5 to 1000 mg per day, or a nasal or inhaled dose of 0.001 to 50 mg per day or 0.01 to 5 mg per day, of the compound 2-[(4*S*)-6-(4-chlorophenyl)-1-methyl-8-(methyloxy)-4*H*-[1,2,4]triazolo[4,3-*a*][1,4]benzodiazepin-4-yl]-*N-*ethylacetamide, calculated as the free base. This amount may be given in a single dose per day or more usually in a number (such as two, three, four, five or six) of sub-doses per day such that the total daily dose is the same. An effective amount of a pharmaceutically acceptable salt thereof, may be determined as a proportion of the effective amount of the compound 2-[(4*S*)-6-(4-chlorophenyl)-1-methyl-8-(methyloxy)-4*H*-[1,2,4]triazolo[4,3-*a*][1,4]benzodiazepin-4-yl]-*N-*ethylacetamide or a pharmaceutically acceptable salt thereof *per se.*

For use in the above therapy the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof may be employed alone or in combination with further therapeutic agents. Therefore in a further aspect there is provided a combination pharmaceutical product comprising 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide or a pharmaceutically acceptable salt thereof together with one or more further therapeutically active agents suitable for use in the treatment of small cell lung cancer.

Examples of such further therapeutic agents are described in Cancer Principles and Practice of Oncology by V.T. Devita and S. Hellman (editors), 6th edition (2001), Lippincott Williams & Wilkins Publishers. A person of ordinary skill in the art would be able to discern which combinations of agents would be useful based on the particular characteristics of the drugs and the cancer involved. Further therapeutic agents to be used in combination with the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof include, but are not limited to, anti-microtubule agents (such as diterpenoids and vinca alkaloids); platinum coordination complexes; alkylating agents (such as nitrogen mustards, oxazaphosphorines, alkylsulfonates, nitrosoureas, and triazenes); antibiotic agents (such as anthracyclins, actinomycins and bleomycins); topoisomerase II inhibitors (such as epipodophyllotoxins); antimetabolites (such as purine and pyrimidine analogues and anti-folate compounds); topoisomerase I inhibitors (such as camptothecins; hormones and hormonal analogues); signal transduction pathway inhibitors (such as tyropsine receptor inhibitors); non-receptor tyrosine kinase angiogenesis inhibitors; immunotherapeutic agents; proapoptotic agents; epigenetic or transcriptional modulators (such as histone deacetylase inhibitors) and cell cycle signaling inhibitors.

The compound 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide or a pharmaceutically acceptable salt thereof and the further therapeutically active agent(s) may be administered together or separately and, when administered separately, this may occur separately or sequentially in any order (by the same or by different routes of administration). The amounts of 2-[(4*S*)-6-(4-chlorophenyl)-1-methyl-8-(methyloxy)-4*H*-[1,2,4]triazolo[4,3-*a*][1,4]benzodiazepin-4-yl]-*N-*ethylacetamide or a pharmaceutically acceptable salt thereof and the further therapeutically active agent(s) and the relative timings of administration will be selected in order to achieve the desired combined therapeutic effect.

The following examples illustrate the invention.

### A pharmaceutical formulation of 2-[(4S)-6-(4-chlorophenyl)-1-methyl-8-(methyloxy)-4H-[1,2,4]triazolo[4,3-a][1,4]benzodiazepin-4-yl]-N-ethylacetamide suitable for oral administration

Representatives formulations comprising 2-[(4*S*)-6-(4-chlorophenyl)-1-methyl-8-(methyloxy)-4*H*-[1,2,4]triazolo[4,3-*a*][1,4]benzodiazepin-4-yl]-*N*-thylacetamide (referenced as Compound A) for use in this invention are shown in the table below.

| **Component** | **Quantity mg/tablet (%w/w)** | | | |
|---|---|---|---|---|
| **Tablet Strength** | **1** | **10** | **30** | **100** |
| Compound A | 1 (1%) | 10 (10%) | 30 (10%) | 100 (20%) |
| Microcrystalline | 94.5 | 85.5 | 256.5 | 376.5 |
| cellulose | (94.5%) | (85.5%) | (85.5%) | (75.3%) |
| Croscarmellose sodium | 4 (4%) | 4 (4%) | 12 (4%) | 20 (4%) |
| Magnesium stearate | 0.5 (0.5%) | 0.5 (0.5%) | 1.5 (0.5%) | 2.5 (0.5%) |
| Colloidal silicon dioxide | - | - | - | 1 (0.2%) |
| Total tablet weight (mg) | 100 | 100 | 300 | 500 |

### Biological Test Methods

### Cell Growth Assay

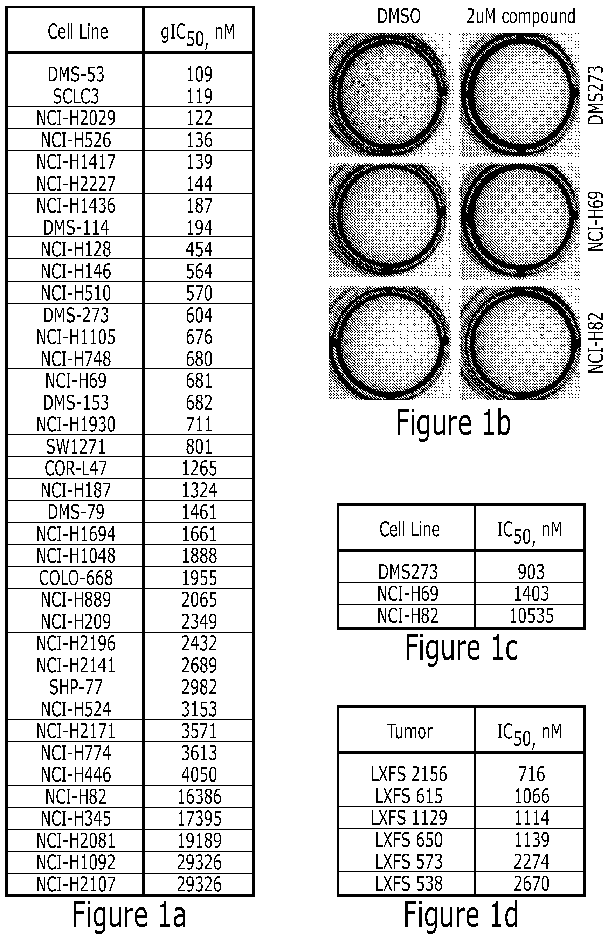

The compound 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide was tested in a 6 day proliferation assay by analogous methods to those described in patent application WO2011054845 using a panel of 38 small cell lung cancer lines. The compound was found to have a gIC₅₀ in the range 109 - 29326 nM (median 1461 nM), with 33 of the 38 lines showing gIC₅₀ < 10000 nM (see Figure 1a).

These data demonstrate that 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide inhibited cell growth in the great majority of small cell lung cancer lines.

### Soft agar SCLC colony formation assay

SCLC cells were plated in soft agar medium at low density and cultured in the presence of the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide for 21 days, after which the colony formation ability was analyzed by microscopy. These data are provided in Figures 1b-d which demonstrates that the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide attenuates colony formation ability of SCLC cell lines and patient-derived SCLC models.

### Caspase 3/7 activation assay

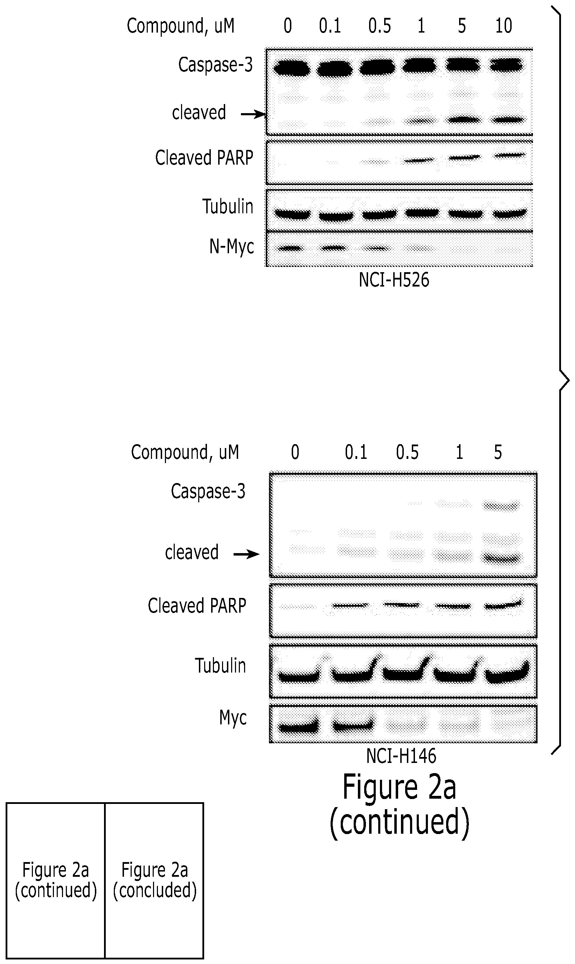

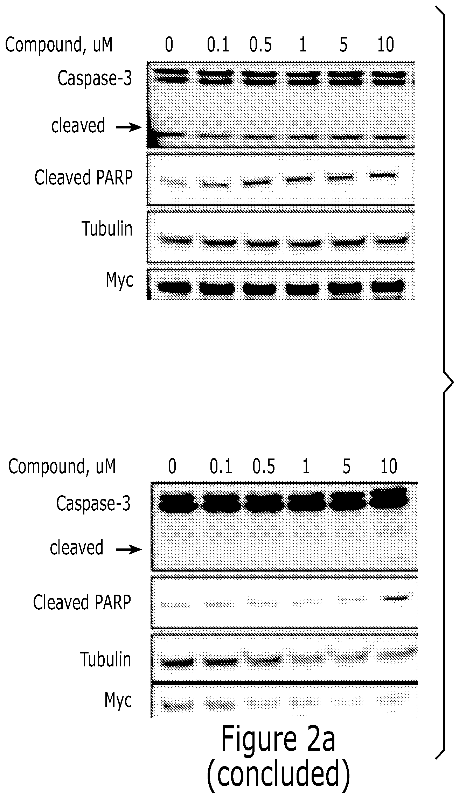

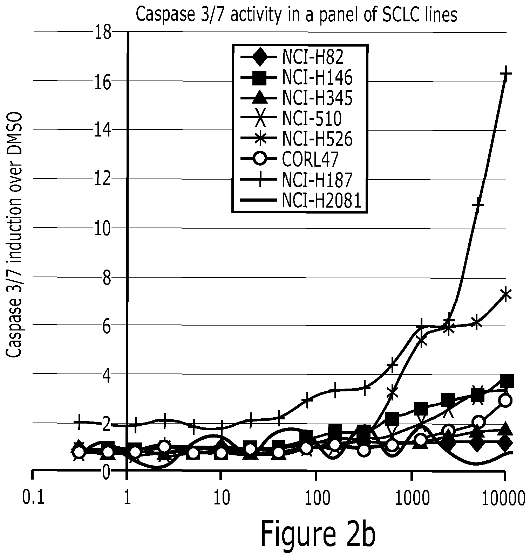

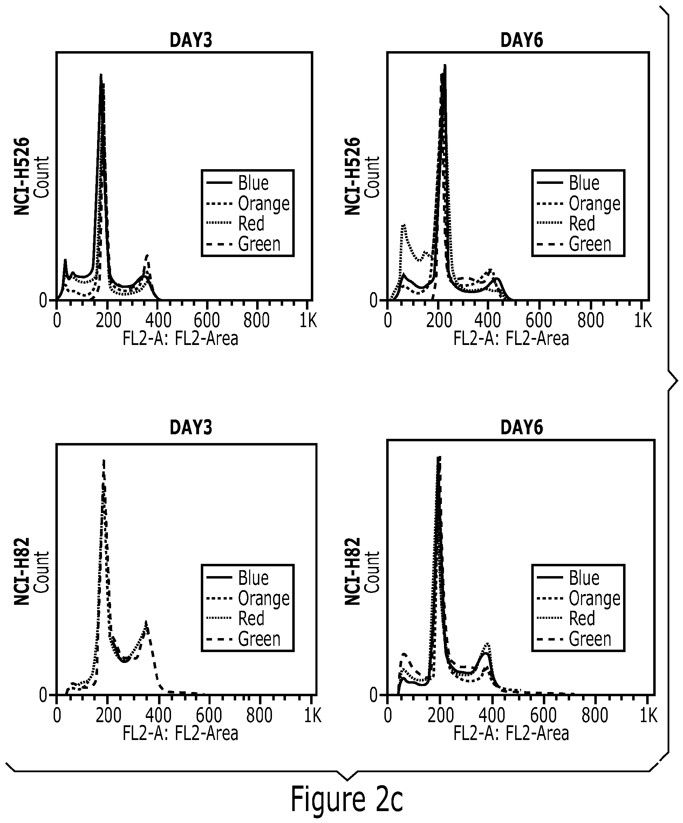

One of the molecular mechanisms of BET inhibition in SCLC models is the induction of caspase 3/7 activity, the subsequent PARP cleavage and the accumulation of cells in subG1 phase of cell cycle. Cells were treated with DMSO or the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide for 3 days, and cell lysates were analysed by immunoblot using antibodies that recognize caspase-3/cleaved caspase-3 and cleaved PARP. The data obtained is shown in Figure 2 with Western blot analysis of Caspase 3/7 activation and PARP cleavage (see Figure 2a), caspase 3/7 activity assay in a panel of SCLC lines 3 days post treatment (see Figure 2b) and propidium iodide staining/FACS analysis of cells treated with the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide (see Figure 2c).

These data indicate that the compound 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide induces cell death in SCLC cell lines.

### SCLC mouse xenograft model

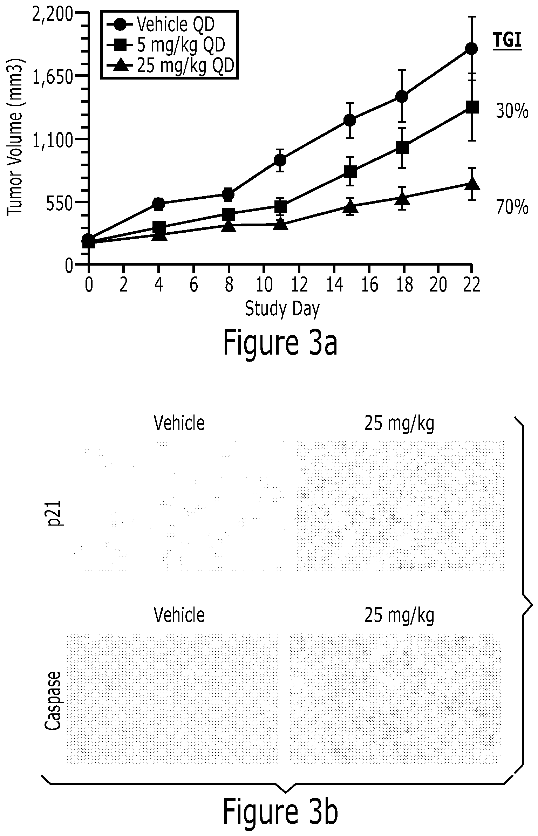

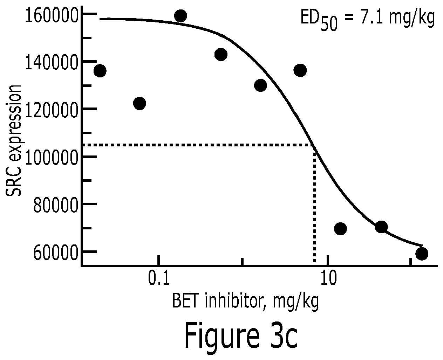

Growth attenuation and the induction of cell death pathways were also observed in an *in vivo* SCLC subcutaneous mouse xenograft model (NCI-H526 cell line), where treatment with 25 mg/kg QD of the compound 2-[(4*S*)-6-(4-chlorophenyl)-1-methyl-8-(methyloxy)-4*H-*[1,2,4]triazolo[4,3-*a*][1,4]benzodiazepin-4-yl]-*N*-ethylacetamide reduced tumour growth by 70 % (see Figure 3a), and was shown to induce p21 protein expression and caspase activation as measured by an antibody-based immunochemistry assay (see Figure 3b). These data are consistent with the *in vitro* data. Dose-dependent changes in target (SRC) gene expression was also observed (see Figure 3c) in a single-dose PK/PD experiment in the SCLC xenograft model (NCI-H526). The dose response measured, with ED₅₀ = 7.1 mg/kg, provides pre-clinical data for projecting a biologically-effective dose in human patients.

## Claims

1. The compound 2-[(4*S*)-6-(4-chlorophenyl)-1-methyl-8-(methyloxy)-4*H-*[1,2,4]triazolo[4,3-*a*][1,4]benzodiazepin-4-yl]-N-ethylacetamide or a pharmaceutically acceptable salt thereof for use in the treatment of small cell lung cancer.

2. The compound for use according to claim 1 which is in the form of a free base.

3. A pharmaceutical composition comprising 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof for use in the treatment of small cell lung cancer.

4. A pharmaceutical composition for use according to claim 3 which is adapted for oral administration.

5. A combination pharmaceutical product comprising 2-[(4*S*)-6-(4-chlorophenyl)-1-methyl-8-(methyloxy)-4*H*-[1,2,4]triazolo[4,3-*a*][1,4]benzodiazepin-4-yl]-*N*-ethylacetamide or a pharmaceutically acceptable salt thereof as defined in claim 1 or claim 2 together with one or more further therapeutically active agents for use in the treatment of small cell lung cancer.

## Patentansprüche

1. Verbindung 2-[(4S)-6-(4-Chlorphenyl)-1-methyl-8-(methyloxy)-4H-[1,2,4]triazolo[4,3-a][1,4]benzodiazepin-4-yl]-N-ethylacetamid oder ein pharmazeutisch annehmbares Salz davon zur Verwendung bei der Behandlung von kleinzelligem Lungenkrebs.

2. Verbindung zur Verwendung gemäss Anspruch 1, die in Form der freien Base vorliegt.

3. Pharmazeutische Zusammensetzung, die 2-[(4S)-6-(4-Chlorphenyl)-1-methyl-8-(methyloxy)-4H-[1,2,4]triazolo[4,3-a][1,4]benzodiazepin-4-yl]-N-ethylacetamid oder ein pharmazeutisch annehmbares Salz davon umfasst, zur Verwendung bei der Behandlung von kleinzelligem Lungenkrebs.

4. Pharmazeutische Zusammensetzung zur Verwendung gemäss Anspruch 3, die zur oralen Verabreichung geeignet ist.

5. Pharmazeutisches Kombinationsprodukt, das 2-[(4S)-6-(4-Chlorphenyl)-1-methyl-8-(methyloxy)-4H-[1,2,4]triazolo[4,3-a][1,4]benzodiazepin-4-yl]-N-ethylacetamid oder ein pharmazeutisch annehmbares Salz davon, wie in Anspruch 1 oder Anspruch 2 definiert, zusammen mit einem oder mehreren therapeutischen Wirkstoffen umfasst, zur Verwendung bei der Behandlung von kleinzelligem Lungenkrebs.

## Revendications

1. Composé 2-[(4*S*)-6-(4-chlorophényl)-1-méthyl-8-(méthyloxy)-4*H*-[1,2,4]triazolo[4,3-*a*][1,4]-benzodiazépin-4-yl]-*N*-éthylacétamide ou l'un de ses sels pharmaceutiquement acceptables pour une utilisation dans le traitement du cancer du poumon à petites cellules.

2. Composé pour une utilisation selon la revendication 1 qui est sous la forme d'une base libre.

3. Composition pharmaceutique comprenant du 2-[(4*S*)-6-(4-chlorophényl)-1-méthyl-8-(méthyloxy)-4*H-*[1,2,4]triazolo[4,3-*a*][1,4]benzodiazépin-4-yl]-*N*-éthyl-acétamide ou l'un de ses sels pharmaceutiquement acceptables pour une utilisation dans le traitement du cancer du poumon à petites cellules.

4. Composition pharmaceutique pour une utilisation selon la revendication 3 qui est adaptée pour une administration orale.

5. Produit pharmaceutique combiné comprenant du 2-[(4*S*)-6-(4-chlorophényl)-1-méthyl-8-(méthyloxy)-4*H-*[1,2,4]triazolo[4,3-*a*][1,4]benzodiazépin-4-yl]-*N*-éthyl-acétamide ou l'un de ses sels pharmaceutiquement acceptables tel que défini dans la revendication 1 ou la revendication 2 conjointement avec un ou plusieurs autres agents thérapeutiquement actifs pour une utilisation dans le traitement du cancer du poumon à petites cellules.
